(19) Europäisches Patentamt / European Patent Office / Office européen des brevets

(11) **EP 4 644 906 A1**

(12) **EUROPEAN PATENT APPLICATION**
published in accordance with Art. 153(4) EPC

(43) Date of publication:
**05.11.2025 Bulletin 2025/45**

(21) Application number: **23912302.9**

(22) Date of filing: **28.12.2023**

(51) International Patent Classification (IPC):
**G01N 33/86** (2006.01)

(52) Cooperative Patent Classification (CPC):
**G01N 33/86**

(86) International application number:
**PCT/JP2023/047188**

(87) International publication number:
**WO 2024/143525 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022212339**

(71) Applicants:
• **Sekisui Medical Co., Ltd.**
**Tokyo 1030027 (JP)**
• **Public University Corporation**
**Nara Medical University**
**Nara 6348521 (JP)**

(72) Inventors:
• **KAWABE, Toshiki**
**Tokyo 103-0027 (JP)**
• **ONISHI, Kengo**
**Tokyo 103-0027 (JP)**
• **NOGAMI, Keiji**
**Kashihara-shi, Nara 634-8521 (JP)**
• **OGIWARA, Kenichi**
**Kashihara-shi, Nara 634-8521 (JP)**
• **SHIMONISHI, Naruto**
**Kashihara-shi, Nara 634-8521 (JP)**

(74) Representative: **Wächtershäuser & Hartz**
**Patentanwaltspartnerschaft mbB**
**Weinstraße 8**
**80333 München (DE)**

(54) **METHOD FOR ANALYZING BLOOD COAGULATION CAPABILITY OF BLOOD SPECIMEN**

(57) A method for analyzing a blood coagulation ability of a blood specimen. The method includes: 1) detecting a coagulation reaction end point Pe of a coagulation reaction curve of a subject blood specimen in which presence or absence of a substance having coagulation factor VIII-substituting activity is required to be detected; 2) calculating T(X), where T(X) represents a measurement point or time at which the coagulation reaction curve reaches X% of Pe, and X is a variable greater than 0 and less than or equal to 100; and 3) detecting the subject blood specimen which contains the substance having coagulation factor VIII-substituting activity, based on T(X).

Fig. 3A

ACQUIRE P(i) — S1

CALCULATE APTT FROM P(i) — S2

COMPARE APTT WITH UPPER LIMIT OF REFERENCE RANGE — S3 → APTT > UPPER LIMIT

APTT ≤ UPPER LIMIT

CALCULATE T(X) FROM P(i) — S4

CALCULATE ONE OR MORE PARAMETERS FROM T(X) — S5

COMPARE PARAMETER(S) WITH THRESHOLD — S6 → NONE SATISFY THRESHOLD

AT LEAST ONE SATISFIES THRESHOLD

OUTPUT APTT CALCULATION RESULT TO WHICH FVIII-SUBSTITUTING SUBSTANCE DETECTION INFORMATION IS ADDED — S7

NORMAL OUTPUT OF APTT CALCULATION RESULT — S8

**Description**

Technical Field

The present invention relates to a method for analyzing a blood coagulation ability of a blood specimen.

Background Art

Hemophilia A is a hemorrhagic disease caused by deficiency or dysfunction of coagulation factor VIII (FVIII). Hemophilia A is classified into congenital hemophilia A with congenital deficiency of FVIII, and acquired hemophilia A caused by autoantibody (inhibitor) to FVIII. Some patients with congenital hemophilia A have inhibitors. Hemophilia A is classified into mild, moderate, and severe depending on the FVIII activity level in the blood. Specimens derived from patients with hemophilia A have a prolonged blood coagulation time in a blood coagulation test depending on the severity.

Emicizumab, which is a hemophilia A treatment, is a recombinant humanized bispecific antibody which can promote the blood coagulation reaction. Emicizumab can bind activated coagulation factor IX (FIXa) at one antigen-binding site, bind coagulation factor X (FX) at the other antigen-binding site, and substitute for the cofactor function of activated coagulation factor VIII (FVIIIa), thereby allowing the blood coagulation reaction to be promoted.

Emicizumab is regularly administered to patients with hemophilia A for the purpose of suppressing the bleeding tendency of patients with hemophilia **A.** The administered emicizumab remains in the blood for a certain period, during which a prolonged blood coagulation time, i.e., a typical characteristic of coagulation abnormality, is not observed in a blood coagulation test of a blood specimen obtained by drawing blood from a patient with hemophilia A to whom emicizumab has been administered. Accordingly, the result shows as if there were no coagulation abnormality. Meanwhile, there is a possibility that reduction in prolonged coagulation time of a patient with hemophilia A (to the reference range upper limit or less) by emicizumab poses a significant danger to the patient. For example, in a case where a patient with hemophilia A undergoes emergency transport and a surgical operation due to a traffic accident or the like, and no prolonged coagulation time (activated partial thromboplastin time: APTT) is observed in a typical preoperative blood coagulation test, a doctor who does not know that the patient has hemophilia A cannot find the patient's reduction in coagulation ability and cannot administer an appropriate treatment. Accordingly, a risk of serious intraoperative bleeding occurs.

Patent Literature 1 describes a method for evaluating a blood coagulation reaction with a substance having a FVIII-substituting activity, by adding a blood coagulation initiation reagent containing activated coagulation factor XI to a blood specimen, and measuring the amount of generated thrombin. Patent Literatures 2 and 3 describe a method for evaluating a coagulation ability of a blood specimen, the method including: acquiring a parameter about the derivative of a coagulation waveform about the blood specimen to which a substance having a FVIII-substituting activity has been administered; acquiring a FVIII activity as a value indicating the coagulation ability from the parameter; and evaluating the coagulation ability of the blood specimen, based on the acquired FVIII activity.

Citation List

Patent Literature

> Patent Literature 1: WO 2014/050926
> Patent Literature 2: WO 2016/170944
> Patent Literature 3: JP-A-2017-106925

Summary of Invention

Technical Problem

The present invention provides a method for analyzing a blood coagulation ability of a blood specimen, the method allowing detecting the blood specimen to which a drug having FVIII-substituting activity has been administered.

Solution to Problem

The present invention provides the following.

[1] A method for analyzing a blood coagulation ability of a blood specimen, the method comprising:

1) detecting a coagulation reaction end point Pe of a coagulation reaction curve of a subject blood specimen in which presence or absence of a substance having coagulation factor VIII-substituting activity is required to be detected;
2) calculating T(X), where T(X) represents a measurement point or time at which the coagulation reaction curve reaches X% of Pe, and X is a variable greater than 0 and less than or equal to 100; and
3) detecting the subject blood specimen which contains the substance having coagulation factor VIII-substituting activity, based on T(X).

[2] The method according to [1], wherein the subject blood specimen is a blood specimen with no prolonged activated partial thromboplastin time (APTT).

[3] The method according to [1] or [2],

wherein the 3) comprises calculating a parameter based on T(X), and detecting the subject blood specimen which contains the substance having coagulation factor VIII-substituting activity, based on the parameter, and
the parameter is at least one selected from the group consisting of an amount of change of T(X), a ratio of T(X), an interval change rate of T(X), and a parameter about a coefficient of variation of T(X) in a given range of X, and standard deviation indices (SDI) thereof.

[4] The method according to [3],

wherein the amount of change of T(X) in the given range of X is $[(T(X_2)/C) - (T(X_1)/C)]$,
the ratio of T(X) in the given range of X is $[T(X_2)/T(X_1)]$, and
the interval change rate of T(X) in the given range of X is $[(X_2 - X_1)/\{(T(X_2)/C) - (T(X_1/C)\}]$,
where $X_1$ and $X_2$ independently range from 1 to 100, $X_2 > X_1$, and C is a constant.

[5] The method according to [3],

wherein the parameter about the coefficient of variation of T(X) is $CV(X_1:X_2)$, $CV(X_1 - J:X_1 + J)$, or $AVE\{CV(X_1 \pm J):CV(X_2 \pm J)\}$,
where
$CV(X_1:X_2)$ is the coefficient of variation of T(X) which is in a range of X from $X_1$ to $X_2$ and is represented by the following equation,

$$CV(X_1:X_2) = SD\{T(X_1:T(X_2)\}/AVE\{T(X_1):T(X_2)\} \times 100 \, [\%],$$

$AVE\{T(X_1):T(X_2)\}$: an average of $T(X_1)$ to $T(X_2)$,
$SD\{T(X_1):T(X_2)\}$: a standard deviation of $T(X_1)$ to $T(X_2)$,
$CV(X_1 - J:X_1 + J)$ is a coefficient of variation of T(X) in a range from $(X_1 - J)$ to $(X_1 + J)$, and
$AVE\{CV(X_1 \pm J):CV(X_2 \pm J)\}$ is an average of $CV(X - J:X + J)$ with X ranging from $X_1$ to $X_2$,
where $X_1$ and $X_2$ independently range from 1 to 100, $X_2 > X_1$, and J = 2 to 44.

[6] The method according to any one of [3] to [5], further comprising detecting the subject blood specimen which contains the substance having coagulation factor VIII-substituting activity, by comparing the parameter based on T(X) with a given threshold.

[7] The method according to any one of [1] to [6], further comprising outputting a result of the detecting.

[8] The method according to any one of [1] to [7], further comprising:

measuring the activated partial thromboplastin time (APTT) of a blood specimen; and
selecting the blood specimen with no prolonged APTT, as the subject blood specimen in which the presence or absence of a substance having coagulation factor VIII-substituting activity is required to be detected.

[9] The method according to any one of [1] to [8], wherein the substance having coagulation factor VIII-substituting activity is a bispecific antibody substituting for a cofactor function of coagulation factor VIII.

[10] The method according to any one of [1] to [9], wherein the substance having coagulation factor VIII-substituting activity is emicizumab.

[11] A program for implementing the method according to any one of [1] to [10].

[12] A device for implementing the method according to any one of [1] to [10].

[13] A system for implementing the method according to any one of [1] to [10], the system comprising: a program for implementing the method according to any one of [1] to [10]; and a device controlled by the program.

[14] The device according to [12] or the system according to [13], further comprising an output unit for outputting a result of the detecting of the subject blood specimen which contains the substance having coagulation factor VIII-substituting activity by the method according to any one of [1] to [10].

Advantageous Effect of Invention

The present invention can detect that a subject specimen is a blood specimen to which a drug having FVIII-substituting activity, such as emicizumab, has been administered, among blood specimens in which no coagulation abnormality can be detected by conventional APTT measurement, using measured data (coagulation reaction data) of a coagulation reaction acquired during APTT measurement.

Brief Description of Drawings

Fig. 1 illustrates an example of a coagulation reaction curve.

Fig. 2 illustrates T(X). Time points at which Pe on the coagulation reaction curve reaches 10%, 50%, and 90% are respectively indicated as T(10), T(50), and T(90).

Fig. 3A illustrates an example of a blood coagulation analyzing process by a method of the present invention.

Fig. 3B is a conceptual diagram illustrating a configuration of a device for performing a method for analyzing a blood coagulation ability by the present invention.

Fig. 4 illustrates a coagulation reaction curve P(i) of a specimen group used in Example. A to C: P(i) of NE, E1, and E2. D and E: illustration of the averages (aE1 and aE2; dotted lines) of P(i) of E1 and E2 together with the average (aNE; solid line) of P(i) of NE.

Fig. 5 illustrates APTT of specimen groups used in Example. NE: control specimen group, E1: specimen derived from a patient with hemophilia A to whom emicizumab has been administered. E2: specimen which contains an inhibitor and has been derived from a patient with hemophilia A to whom emicizumab has been administered.

Fig. 6 illustrates corrected reaction curves of specimen groups used in Example. A to C: corrected reaction curves of NE, E1, and E2. D and E: illustration of the averages (aE1 and aE2; dotted lines) of the corrected reaction curves of E1 and E2 together with the average (aNE; solid line) of the corrected reaction curve of NE.

Fig. 7 illustrates A: a plot of the interval change rate of T(X) of each specimen against APTT. B: **a** distribution of the interval change rate of each specimen group.

Fig. 8 illustrates A: a plot of the amount of change of T(X) of each specimen against APTT. B: a distribution of the amount of change of T(X) of each specimen group.

Fig. 9 illustrates A: a plot of T70/T30 of each specimen against APTT, B: distributions of T70/T30 of each specimen group.

Fig. 10 illustrates CV curves of specimen groups used in Example. A to C: CV curves of NE, E1, and E2. D and E: illustration of the averages (aE1 and aE2; dotted lines) of the CV curves of E1 and E2 together with the average (aNE; solid line) of the CV curve of NE.

Fig. 11 illustrates A: a plot of CV(30:50) of each specimen against APTT, B: a distribution of CV (30:50) of each specimen group.

Fig. 12 illustrates A: a plot of AVE{CV(30 $\pm$ 4):CV(50 $\pm$ 4)} of each specimen against APTT, B: a distribution of AVE {CV(30 $\pm$ 4):CV(50 $\pm$ 4)} of each specimen group.

Fig. 13 illustrates A: a plot of Vmax of each specimen against APTT, and B: a distribution of Vmax of each specimen group.

Fig. 14 illustrates A: a plot of VmaxT of each specimen against APTT, and B: a distribution of VmaxT of each specimen group.

Description of Embodiments

All Patent Literatures, Non Patent Literatures, and other publications cited in the present Description are incorporated herein by reference in their entirety.

In a blood coagulation test for measuring the activated partial thromboplastin time (APTT), a predetermined reagent is added to a subject blood specimen, a subsequent blood coagulation reaction is measured, and the blood coagulation time (APTT) is measured from the coagulation reaction data. To measure the blood coagulation reaction, general means is used; for example, optical means for measuring the scattered light intensity, transmittance, and absorbance, or mechanical means for measuring the viscosity of plasma are used. Typically, the blood coagulation reaction is represented by a coagulation reaction curve which indicates the temporal change in the amount of coagulation reaction. In the following Description, the blood specimen, blood coagulation reaction, and blood coagulation time are sometimes simply called the specimen, coagulation reaction, and coagulation time, respectively.

With specimens having a coagulation abnormality factor, APTT is often prolonged in comparison with that of a normal specimen with no coagulation abnormality. The prolongation of APTT is one of major indicators for the coagulation abnormality related to a bleeding risk or a thrombosis risk. With a specimen derived from a patient with hemophilia A, APTT is typically prolonged beyond a reference range (a range allowing a specimen having APTT within this range to be regarded "normal"). Meanwhile, in a case where a substance having FVIII-substituting activity (hereinafter, also called "FVIII-substituting substance"), such as emicizumab, has been administered to a hemophilia A specimen, APTT of a specimen of the patient is not prolonged, and falls within the reference range or is reduced below the reference range. It is difficult to distinguish whether the subject specimen is a specimen derived from a hemophilia A specimen to which the FVIII-substituting substance has been administered or not based on the reference range of APTT.

The present inventors found that even in a case where no prolonged coagulation time is observed by measuring APTT, the blood specimen derived from a patient with hemophilia A to whom emicizumab has been administered exhibits a different progression of the blood coagulation reaction (blood coagulation reaction curve) from that of a specimen to which no emicizumab has been administered.

In the present invention, presence or absence of FVIII-substituting substance in the subject specimen is detected based on coagulation reaction data of the subject specimen measured during APTT measurement, specifically, a parameter derived from a coagulation reaction curve. The parameter used in the present invention is a parameter obtained without differentiating the coagulation reaction curve (coagulation waveform), and is different from a parameter about the derivative of the coagulation waveform as used in evaluation of the coagulation ability in Patent Literatures 2 and 3 described above. In the present invention, among specimens in which no coagulation abnormality is detected by conventional APTT measurement, a specimen to which the FVIII-substituting substance has been administered can be detected.

In the present Description, the FVIII-substituting substance refers to a substance that can substitute for an FVIII cofactor function. The FVIII cofactor function refers to a function of activated FVIII (FVIIIa) forming a complex of activated coagulation factor IX (FIXa) and coagulation factor X (FX). An example of the FVIII-substituting substance may be a bispecific antibody substituting for the FVIII cofactor function (for example, a bispecific antibody simulating the FVIII structure). An example of such a bispecific antibody may be emicizumab. Emicizumab is a recombinant humanized bispecific antibody that binds the activated coagulation factor IX (FIXa) at one antigen-binding site, and binds the coagulation factor X (FX) at the other antigen-binding site, and substitutes for the FVIII cofactor function. Emicizumab is a drug for suppressing the bleeding tendency of congenital and acquired hemophilia A. Furthermore, next-generation FIXa/FX bispecific antibodies (Mim8, NXT007 and the like) having an action mechanism similar to that of emicizumab have also been developed.

[Method for analyzing blood coagulation ability of blood specimen]

The method for analyzing a blood coagulation ability of a blood specimen in the present invention (hereinafter also called the method of the present invention) can detect the difference between the coagulation abilities of blood specimens which cannot be discriminated based on the coagulation time (APTT), as the difference in progression of the coagulation reaction (coagulation reaction curve). In the present invention, based on the coagulation reaction curve of the subject specimen, a parameter for analyzing the blood coagulation ability of the subject specimen is acquired without the need to obtain the differential curve.

The method of the present invention is described below.

1. Acquisition of coagulation reaction curve and detection of coagulation reaction end point Pe.

1.1. Coagulation reaction measurement

Preferably, the method of the present invention uses, as the subject specimen, the plasma of a subject drawn for a coagulation test. An anticoagulant typically used for a coagulation test may be added to the specimen. For example, blood is drawn using a blood collection tube which contains sodium citrate, and is subsequently centrifugally separated, thus obtaining plasma.

The coagulation reaction curve of a subject specimen used for the method of the present invention is calculated from coagulation reaction data acquired in accordance with procedures for measuring the coagulation reaction in typical APTT measurement. Specifically, in coagulation reaction measurement, an APTT measurement reagent is added to the subject specimen, and the blood coagulation reaction is started. The coagulation reaction in a mixture containing the reagent and the subject specimen is measured. APTT measurement reagents are commercially available (for example, APTT reagent Coagpia APTT-N, and Coagpia APTT-N calcium chloride liquid, which are manufactured by Sekisui Medical Co., Ltd.). To measure the coagulation reaction, general means is used; for example, optical means for measuring the scattered light intensity, transmittance, absorbance or the like, or mechanical means for measuring the viscosity of blood are used. In the following present Description, the method of the present invention is described using, as an example, coagulation reaction measurement based on the scattered light intensity.

The reaction initiation time point of the coagulation reaction can typically be defined as a time point at which the reagent is mixed into the specimen and the coagulation reaction is started. Alternatively, other timing may be defined as a reaction initiation time point. The duration of measuring the coagulation reaction can range, for example, from several tens of seconds to about seven minutes from the time point of mixing the specimen and the reagent. The measurement time may be a freely defined fixed value, or may be up to a time point at which the end of the coagulation reaction of each specimen is detected. During the measurement time, measurement of the progress of the coagulation reaction (measurement of the scattered light intensity) can be repeatedly performed at predetermined intervals. For example, measurement may be performed at intervals of 0.1 seconds. The temperature of the mixture during measurement is under a typical condition, for example, between 30°C and 40°C, inclusive, preferably, between 35°C and 39°C, inclusive. Various conditions of measurement can be set in conformity with the subject specimen, reagent, measurement means and the like as appropriate.

A series of operations in the coagulation reaction measurement described above can be performed using an automatic analysis device. An example of the automatic analysis device may be a blood coagulation automatic analysis device CP3000 (manufactured by Sekisui Medical Co., Ltd). Alternatively, some operations may be manually performed. For example, preparation of the subject specimen can be performed by a person, and operations thereafter can be performed by the automatic analysis device.

1.2. Acquisition of coagulation reaction curve

A coagulation reaction curve P(i) is acquired from the measured coagulation reaction data. Here, "i" denotes the number of measurement points or time from the coagulation reaction initiation (also simply called the number of measurement points, and time, respectively). For example, in the case where a measurement (photometry) interval is 0.1 seconds, it is represented that time = 0.1 × the number of measurement points. That is, P(i) may be a function of the number of measurement points, or a function of time. In the following present Description, P(i) is sometimes simply abbreviated as P. Typically, the coagulation reaction curve P is obtained by applying a denoising or smoothing process to measurements of the coagulation reaction measurement by typical means, or applying zero adjustment for adjusting the initial value or curve relativizing as required.

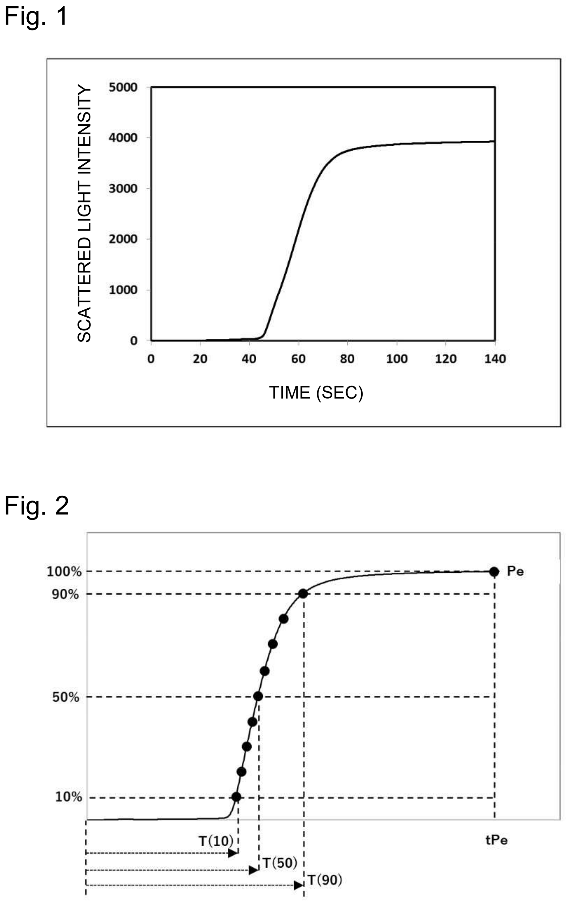

Fig. 1 illustrates an example of the coagulation reaction curve. The abscissa axis of Fig. 1 indicates time, and the ordinate axis indicates the coagulation reaction (scattered light intensity), which are adjusted such that the scattered light intensity at the measurement initiation time point is zero. The coagulation reaction of the mixture proceeds over time. Thus, the scattered light intensity increases. The coagulation reaction curve based on the scattered light intensity as illustrated in Fig. 1 is typically sigmoidal.

From the coagulation reaction curve P, its differential curve, for example, the reaction rate curve (first derivative) or the reaction acceleration curve (second derivative), may be acquired if required. The differential process for the coagulation reaction curve can be executed by any method, and can be performed by, for example, calculating the intra-interval average slope. In the present Description, the coagulation reaction curve, the first derivative curve such as the reaction rate curve, and the second derivative curve such as the reaction acceleration curve are also called "zero-order curve", "first-order curve", and "second-order curve", respectively.

**1.3.** Detection of coagulation reaction end point Pe

The method of the present invention detects the coagulation reaction end point Pe of the coagulation reaction curve P of the subject specimen. Pe can be determined in accordance with any reference, such as a time point at which P reaches a plateau, a time point at which the first derivative curve of P decreases to zero or to a certain value after reaching a peak (see WO 2021/206107), or an earliest time point at which the accumulated ratio of P(i) in a minute time period becomes less than a threshold (for example, 1.001) (see WO 2021/132552 or after-mentioned Example). The detection of Pe may be performed after P is acquired before a predetermined measurement time elapses. Alternatively, Pe may be detected in parallel with the acquisition of P, and upon detection of Pe, the acquisition of P may be finished. In the latter case, the measurement time for one specimen can be reduced to the required minimum. For example, procedures described in WO 2021/206107 or WO 2021/132552 allow the detection of Pe in parallel with the acquisition of P (what is called the detection of Pe in real time).

As an embodiment of the method of the present invention, an example of a Pe detection procedure based on a method described in WO 2021/132552 is described in detail. The accumulated ratio of P(i) in a minute time period is defined as Z(i), and is calculated by the following equations.

$$\texttt{Accumulated ratio Z(i) = Pb(i)/Pa(i)}$$

$$\texttt{Pa(i) = sum of P(i - m) to P(i - 1)}$$

$$\texttt{Pb(i) = sum of P(i + 1) to P(i + m)}$$

In the above equations, i denotes the measurement point number, and m can be set depending on the measurement condition, analysis items and the like of the coagulation reaction as appropriate, and for example, m = 10 to 30. P(i) at the earliest measurement point or time point when Z(i) becomes less than a threshold Zs is detected as the coagulation reaction end point Pe. While Zs can be set depending on the analysis items as appropriate, it is greater than one and less than or equal to 1.100. For example, in the case of APTT measurement, it is preferable that Zs be less than or equal to 1.050, and more preferably, is in a range from 1.010 to 1.001. To prevent erroneous detection of Pe in an initial response or the like, it is preferable that the calculation of Z(i) be performed after i reach a predetermined calculation initiation point and P(i) reach a predetermined value or greater. In this procedure, while the coagulation reaction is measured, P(i) is acquired and Z(i) is calculated in parallel, thus allowing Pe to be detected.

### 1.4. APTT calculation

The coagulation time (APTT) of the subject specimen can be calculated from the coagulation reaction data acquired by the coagulation reaction measurement. The method of calculating APTT is not specifically limited. For example, APTT can be calculated by any method, based on the P(i) or its first derivative curve. Examples of the APTT calculating method include: a method of calculating, as the coagulation time, a time point at which P(i) reaches N% of the coagulation reaction end point Pe (percent detection method); a method of calculating, as the coagulation time, a time point at which the first-order curve of P reaches N% of the maximum; a method of assuming, as the calculation starting point Te, a time point when the accumulated ratio of P(i) in a minute time period reaches a predetermined value, and calculating, as the coagulation time, a time point at which P(i) reaches N% of P(Te) (see JP-A-H6-249855); a method of calculating the coagulation time based on the temporal change in the accumulated ratio of P(i) in a minute time period (see WO 2021/132552); a method of calculating the coagulation time, based on the weighted average time of the first-order curve of P (see WO 2021/177452); and a method of assuming, as the calculation starting point Te, a time point when the first-order curve of P reaches a predetermined value after reaching the maximum, and calculating, as the coagulation time, a time point at which P(i) reaches N% of P(Te) (see WO 2021/206107).

### 2. Calculation of T(X)

Upon detection of Pe, T(X) which represents the number of measurement points or time at which the coagulation reaction curve P(i) reaches X% of Pe is calculated. More specifically, T(X) is the number of measurement points or time from the measurement initiation point of the coagulation reaction (time 0) until P reaches X% of Pe. That is, the following equation holds for T(X).

$$\texttt{P(T(X)) = Pe × X\%}$$

Here, X is a variable which is greater than 0 and less than or equal to 100. Thus, T(X) is a function of the variable X. In the method of the present invention, it is preferable that X range from 1 to 100, and more preferably, from 5 to 95. For example, X may have values which are each apart by one or more (that is, the increment of X ≥ 1), or values which are each apart by five or more (that is, the increment of X ≥ 5). Preferably, X increases from the initial value by an increment of J (J is an integer ranging from 1 to 5). For example, X is a variable changing by an increment of one in a range from 1 to 100 (that is, X = {1, 2, 3, ..., 97, 98, 99, 100}). In another example, X is a variable changing by an increment of five in a range from 5 to 95 (that is, X = {5, 10, 15, ..., 90, 95}).

Alternatively, T(X) may be represented as an average of T(X - K) to T(X + K). In this case, it is preferable that K be smaller than the increment J of X described above. For example, provided that K = 2, T(X) can be represented as an average of T(8) to T(12) when X = 10, or represented as an average of T(13) to T(17) when X = 15.

Fig. 2 illustrates T(X). The diagram indicates a coagulation reaction curve indicating points reaching 10 to 90% of Pe, and indicates time points reaching 10%, 50%, and 90% of Pe as T(10), T(50), and T(90), respectively. A time point tPe for Pe corresponds to T(100). Note that in the present Description and the drawings, T(X) is sometimes represented as "TX" with representation of parentheses being omitted.

3. Acquisition of parameter for analyzing blood coagulation ability

3.1. Subject specimen in which parameter should be acquired

Next, in the method of the present invention, the parameter for analyzing the blood coagulation ability of the subject specimen is acquired based on the T(X). In the method of the present invention, the parameter may be acquired for the subject specimen in which the presence or absence of the FVIII-substituting substance is required to be detected. Typically, the subject specimen in which the presence or absence of the FVIII-substituting substance is required to be detected is a specimen with no prolonged APTT. Without the need to check for the presence or absence of the FVIII-substituting substance, the specimen with prolonged APTT is estimated as a specimen with a coagulation abnormality (that is, a high bleeding risk or thrombosis risk). Accordingly, application of the method of the present invention is not necessarily required.

In one embodiment of the method of the present invention, the coagulation reaction of any subject specimen is measured, APTT is calculated, and subsequently, a specimen with no prolonged APTT is selected as a subject specimen in which presence or absence of the FVIII-substituting substance is required to be detected. About the selected subject specimen, T(X) can be acquired from the coagulation reaction data (for example, corrected P(i), and Pe) obtained by measuring the coagulation reaction. The T(X) may be calculated together with the P(i) and the like during APTT calculation, or T(X) may be obtained by calculating APTT, selecting a subject specimen with no prolonged APTT to which the method of the present invention should be applied, and then performing calculation based on the coagulation reaction data of the selected subject specimen (for example, corrected P(i), and Pe). In another embodiment of the method of the present invention, from existing coagulation reaction data, a specimen which has been already identified to have no prolonged APTT is selected as a subject specimen in which the presence or absence of the FVIII-substituting substance is required to be detected. From the existing coagulation reaction data, T(X) can be acquired.

3.2. Acquisition of parameter based on T(X)

Based on the T(X), the parameter for analyzing the blood coagulation ability of the subject specimen can be calculated. Examples of the parameter include the amount of change of T(X), the ratio of T(X), and the interval change rate of T(X) in a given range of X. Preferably, the amount of change of T(X) is represented as $[T(X_2) - T(X_1)]$, the ratio of T(X) is represented as $[T(X_2)/T(X_1)]$, and the interval change rate of T(X) is represented as $[(X_2 - X_1)/(T(X_2) - T(X_1))]$. Alternatively, the amount of change of T(X), and the interval change rate of T(X) may be calculated as the amount of change in the relative value of T(X), and the interval change rate. For example, the amount of change of T(X) can be represented as $[(T(X_2)/C) - (T(X_1)/C)]$, and the interval change rate of T(X) can be represented as $[(X_2 - X_1)/\{(T(X_2)/C) - (T(X_1)/C)\}]$. Here, it is preferable that $X_1$ and $X_2$ independently range from 1 to 100, and more preferably, from 5 to 95, where $X_2 > X_1$. More specifically, in the case of ratio of T(X), it is preferable that $X_1 = 5$ to 55, and $X_2 = 30$ to 95, where $X_2 - X_1 = 10$ to 90. In the case of the amount of change of T(X), it is preferable that $X_1 = 5$ to 55, and $X_2 = 35$ to 95, where $X_2 - X_1 = 10$ to 90. In the case of the interval change rate of T(X), it is preferable that $X_1 = 5$ to 55, and $X_2 = 30$ to 90, where $X_2 - X_1 = 10$ to 85. C is a constant, and may be, for example, APTT, VmaxT (time at which the reaction rate is the maximum; see description below), any T(X) in a range of X = 40 to 60 or the like.

Another example of the parameter may be a parameter about the coefficient of variation CV of T(X). In one example, the parameter about the CV is $CV(X_1:X_2)$ of the following equation, and represents the coefficient of variation CV of T(X) in the range of X from $X_1$ to $X_2$. Here, it is preferable that $X_1$ and $X_2$ independently range from 1 to 100, where $X_2 > X_1$.

$$CV(X_1:X_2) = SD\{T(X_1):T(X_2)\}/AVE\{T(X_1):T(X_2)\} \times 100 \; [\%],$$

where

AVE$\{T(X_1):T(X_2)\}$: an average of $T(X_1)$ to $T(X_2)$.
SD$\{T(X_1):T(X_2)\}$: a standard deviation of $T(X_1)$ to $T(X_2)$.

Preferably, $X_1 = 5$ to 55 and $X_2 = 30$ to 95, and $X_2 - X_1 = 10$ to 90.

In another example, the parameter about the CV is $CV(X_1 - J:X_1 + J)$ representing the coefficient of variation of T(X) in a range from $(X_1 - J)$ to $(X_1 + J)$ (also represented as $CV(X_1 \pm J)$). Here, it is preferable that $X_1$ range from 25 to 65, and J range from 2 to 44.

In another example, the parameter about the CV is the average $[AVE\{CV(X_1 \pm J):CV(X_2 \pm J)\}]$ of $CV(X \pm J)$ with X ranging from $X_1$ to $X_2$, preferably, $X_1$ = 10 to 65, $X_2$ = 25 to 95, and $X_2$ - $X_1$ = 5 to 85 (J is as defined above).

A value obtained by converting the value of each parameter described above into the standard deviation index (SDI) with respect to a reference value can be used as the parameter for analyzing the blood coagulation ability of the subject specimen in the present invention. Specifically, the absolute value of SDI (|SDI|) obtained by the following equation using the statistics of the specimen containing no FVIII-substituting substance (that is, a specimen which contains no FVIII-substituting substance and has no prolonged coagulation time; hereinafter also called a non-administered (ND) specimen) can be adopted as the parameter.: |SDI| = |{(parameter value calculated from the subject specimen) - (average of parameter values calculated from ND specimen group)}/(standard deviation of parameter values calculated from the ND specimen group) |.

4. Detection of specimen containing FVIII-substituting substance

As described in the after-mentioned Example, the values of the aforementioned parameters calculated in the present invention for analyzing the blood coagulation ability showed different tendencies between specimens containing the FVIII-substituting substance obtained from patients to whom the FVIII-substituting substance (emicizumab) had been administered, and specimens containing no FVIII-substituting substance (emicizumab). Based on these parameters, the specimen containing the FVIII-substituting substance can be detected.

In the analysis of the blood coagulation ability by the present invention, examples of the parameters used to detect the specimen containing the FVIII-substituting substance may be at least one selected from the group consisting of the amount of change of T(X), the ratio of T(X), and the interval change rate of T(X) in the given range of X described above, and the parameter about the coefficient of the variation of T(X). A more preferable example of the parameter may be at least one selected from the group consisting of the aforementioned $[(T(X_2)/C) - (T(X_1)/C)]$, $[T(X_2)/T(X_1)]$, $[(X_2 - X_1)/\{(T(X_2)/C) - (T(X_1)/C)\}]$, $CV(X_1:X_2)$, $CV(X \pm J)$, and $AVE\{CV(X_1 \pm J):CV(X_2 \pm J)\}$ (here, $X_1$, $X_2$, C, and J are as described above). Alternatively, the SDI of each of the aforementioned parameters may also be an example of the parameter used to detect the specimen containing the FVIII-substituting substance. In the analysis of the blood coagulation ability by the present invention, these parameters may be any one kind of them, or two or more kinds may be combined and used.

In the detection of the specimen containing the FVIII-substituting substance based on the parameter, for example, in a case where the parameter is the interval change rate of $T(X)[(X_2 - X_1)/\{(T(X_2)/C) - (T(X_1)/C)\}]$, the subject specimen can be detected as the specimen containing the FVIII-substituting substance if the parameter calculated from the subject specimen is less than or equal to a predetermined detection threshold (hereinafter, also simply called "threshold") or smaller than the threshold. On the other hand, in a case where the parameter is the amount of change of T(X) $[(T(X_2)/C) - (T(X_1)/C)]$, the ratio of T(X) $[T(X_2)/T(X_1)]$, $CV(X_1:X_2)$, $CV(X \pm J)$, or $AVE\{CV(X_1 \pm J):CV(X_2 \pm J)\}$, the subject specimen can be detected as the specimen containing the FVIII-substituting substance if the parameter calculated from the subject specimen is greater than or equal to the threshold, or greater than the threshold.

A value which can distinguish the specimen containing the FVIII-substituting substance, and the specimen containing no FVIII-substituting substance(ND specimen) from each other can be set as the threshold. For example, in a case where the parameter is $[(X_2 - X_1)/(T(X_2)/C - T(X_1)/C)]$, a value based on the statistics (the minimum, average, standard deviation or the like) of parameters calculated from the ND specimen group (for example, the average - K $\times$ standard deviation (SD), where it is preferable that K range from 1.2 to 2.4) can be set as the threshold. On the other hand, in a case where the parameter is $[T(X_2)/C - T(X_1)/C]$, $[T(X_2)/T(X_1)]$, $CV(X_1:X_2)$, $CV(X \pm J)$, or $AVE\{CV(X_1 \pm J):CV(X_2 \pm J)\}$, a value based on the statistics (maximum, average, standard deviation or the like) of the parameters calculated from the ND specimen group (for example, average + K $\times$ standard deviation (SD), where it is preferable that K range from 1.3 to 3.4) can be set as the threshold.

In the case where the parameter is the SDI of each parameter described above, the threshold can be set based on the SDI of the parameter preliminarily calculated from the specimen containing the FVIII-substituting substance group or the ND specimen group. For example, the threshold for |SDI| described above can be set as follows: what has a value closest to the distribution region of the ND specimen group is extracted from among the parameters calculated from the specimen containing the FVIII-substituting substance group, and its |SDI| is obtained by the aforementioned equation; meanwhile, what has a value closest to the distribution region of the FVIII-substituting substance group is extracted from among the parameters calculated from the ND specimen group, and its |SDI| is obtained by the aforementioned equation; and an intermediate value between the former |SDI| and the latter |SDI| can be set as the threshold (see after-mentioned Example 5). Alternatively, instead of the intermediate value, any value which can distinguish the distributions of the former |SDI| and the latter |SDI| from each other may be set as the threshold. If |SDI| calculated from the subject specimen is greater than the threshold, the subject specimen can be detected as the specimen containing the FVIII-substituting substance.

Two or more of the parameters can be used as the reference for the detection. In this case, a threshold is set for each parameter, and it is determined whether each parameter calculated from the subject specimen satisfies the reference based on the corresponding threshold. In one example, if all the two or more parameters satisfy the threshold, the subject specimen is detected as the specimen containing the FVIII-substituting substance. In another example, if at least one of the two or more parameters satisfies the threshold, it is detected that there is a possibility that the subject specimen is the specimen containing the FVIII-substituting substance.

The specimen detected as the specimen containing the FVIII-substituting substance is a specimen derived from a patient with an abnormality of the blood coagulation ability to which the FVIII-substituting substance has been administered, and the patient is estimated as a patient with a high bleeding risk in surgery or the like. Clinically, emicizumab, which is a drug for suppressing the bleeding tendency of hemophilia A, has been known as an FVIII-substituting substance. Consequently, a patient from whom the specimen containing the FVIII-substituting substance detected by the method of the present invention has been derived can be estimated as a patient with hemophilia A to whom emicizumab has been administered.

As for the aforementioned detection reference, those skilled in the art can understand that as long as the specimen containing the FVIII-substituting substance can be detected, references which are "greater than the threshold" and "the greater than or equal to the threshold" can be interchangeably used, and likewise, references which are "less than the threshold" and "less than or equal to the threshold" can be interchangeably used.

5. Application to other coagulation reaction measurement methods

The method of the present invention has thus been described above using, as the example, the case of coagulation reaction measurement based on the scattered light intensity. However, those skilled in the art can apply other coagulation reaction measurement methods (for example, coagulation reaction measurement based on the transmittance, absorbance, viscosity and the like) to the coagulation reaction measurement in the present invention. Accordingly, such applications are encompassed by the scope of the present invention. For example, in the reaction $P(i)$ represented by an inverted sigmoidal coagulation reaction curve based on the transmittance (transmittance light intensity), the positive and the negative are reversed with respect to that described based on the scattered light intensity. It is obvious to those skilled in the art that in such a case, in the method of the present invention, the sign of $P(i)$ is reversed.

6. Program and device

The method for analyzing the blood coagulation ability in the present invention can be automatically performed using a computer program. Consequently, an aspect of the present invention is a program for performing the aforementioned method for analyzing the blood coagulation ability in the present invention. A series of steps of the aforementioned method of the present invention can be automatically performed by an automatic analysis device. Consequently, an aspect of the present invention is a device for performing the aforementioned method for analyzing the blood coagulation ability in the present invention. Execution of the method of the present invention by the device can be controlled by the program in the present invention. Another aspect of the present invention is a system for performing the method for analyzing the blood coagulation ability in the present invention. The system includes the device or the program for performing the aforementioned method for analyzing the blood coagulation ability in the present invention.

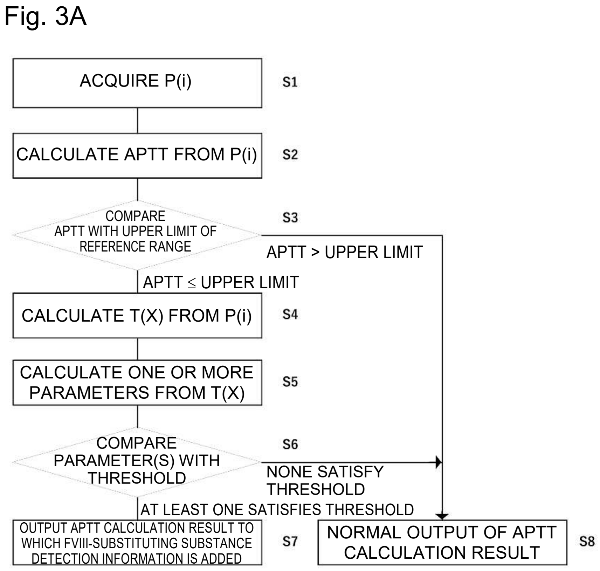

An example of a blood coagulation analyzing process by the present invention executed under control of the program of the present invention is described below and in Fig. 3A.

S1: Acquire $P(i)$ from (freely) measured coagulation reaction data.
S2: Calculate APTT from $P(i)$.
S3: Go to S4 if APTT is not prolonged, and to S8 if APTT is prolonged.
S4: Calculate $T(X)$ from $P(i)$.
S5: Calculate one or more kinds of parameters from $T(X)$.
S6: Compare the parameter(s) with threshold.

$\rightarrow$ If at least one parameter satisfies the threshold, go to S7.
$\rightarrow$ If no parameter satisfies the threshold, go to S8.

S7: Output detection of APTT and the specimen containing the FVIII-substituting substance.
S8: Output APTT.

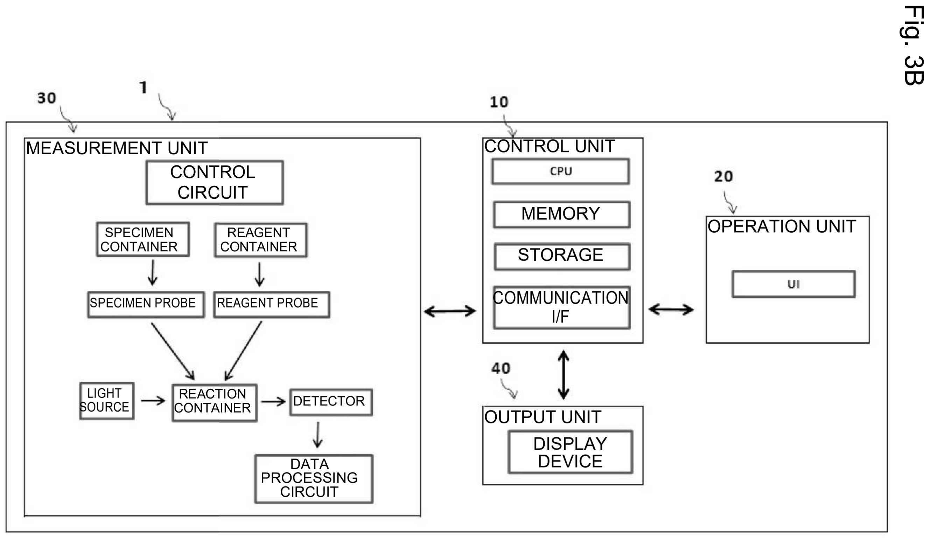

One embodiment of the device in the present invention is described below. The one embodiment of the device of the present invention is an automatic analysis device 1 as illustrated in Fig. 3B. The automatic analysis device 1 includes a control unit 10, an operation unit 20, a measurement unit 30, and an output unit 40.

The control unit 10 controls the entire operation of the automatic analysis device 1. The control unit 10 may be made up of a computer (for example, a personal computer). The control unit 10 includes a CPU, a memory, a storage, a communication interface (I/F) and the like, and can perform processing of commands from the operation unit 20, control of the operation of the measurement unit 30, storing and data analysis of measured data received from the measurement unit 30, storing of an analysis result, control of outputting of the analysis result by the output unit 40 and the like. Furthermore, the control unit 10 may be connected to other devices, such as an external medium, and a host computer. Note that in the control unit 10, the computer which controls the operation of the measurement unit 30, and the computer which analyzes data measured by the unit 30 may be identical to or different from each other.

The operation unit 20 acquires input from an operator, and transmits the obtained input information to the control unit 10. For example, the operation unit 20 includes an user interface (UI), such as a keyboard and a touch panel. Under control of the control unit 10, the output unit 40 outputs: a detection result of the specimen containing the FVIII-substituting substance, and coagulation reaction data measured by the measurement unit 30 as required; and P(i), Pe, T(X), and a parameter for analyzing the blood coagulation ability based thereon, and an analysis result, such as the coagulation time (APTT) as required. For example, the output unit 40 includes a display device, such as a display.

The measurement unit 30 executes the series of operations for the blood coagulation test, and acquires measured data of the coagulation reaction of a sample which contains the blood specimen. The measurement unit 30 includes various instruments and analysis modules required for the blood coagulation test, for example, a specimen container which stores the blood specimen, a reagent container which stores a reagent for a test, a reaction container for the reaction between the specimen and the reagent, a probe for dispensing the blood specimen and the reagent into the reaction container, a light source, a detector for detecting scattered light or transmitted light from the sample in the reaction container, a data processing circuit which transmits data from the detector to the control unit 10, and a control circuit which controls the operation of the measurement unit 30 in response to an instruction of the control unit 10. Alternatively, in a case where P(i), Pe, and T(X) are acquired using known coagulation reaction data, and the parameter is calculated, the measurement unit 30 is not required.

The control unit 10 performs analysis of the coagulation reaction of the specimen, based on the coagulation reaction data. This analysis may include acquisition of the aforementioned P(i) and Pe, calculation of APTT, calculation of T(X) and parameters, and detection of the specimen containing the FVIII-substituting substance using the parameter. Alternatively, P(i), Pe, and T(X) may be created by the control unit 10 based on the measured data from the measurement unit 30, or may be created by another device, for example, the measurement unit 30 and then be transmitted to the control unit 10. The control unit 10 may store a threshold used to detect the specimen containing the FVIII-substituting substance, or the control unit 10 may import the threshold stored in an external device or on a network, for the sake of the detection.

This analysis described above can be executed by the program for performing the method of the present invention. Consequently, the control unit 10 may include the program for performing the method for analyzing the blood coagulation ability in the present invention.

The analysis result in the control unit 10 is transmitted to the output unit 40 and is output. The output may be in any form, such as being displayed on a screen, being transmitted to a host computer, or being printed. Output information from the output unit can include P(i), Pe, APTT, T(X), a parameter for analyzing the blood coagulation ability, and a detection result of the specimen containing the FVIII-substituting substance. Furthermore, the output information may include other information obtained by APTT measurement (detection of an initial response, possibility of a milky specimen and the like). The kind of output information from the output unit can be controlled by the program of the present invention.

Examples

The present invention is described in further detail below with reference to Examples. However, the present invention is not limited to these Examples. The coagulation reaction measurement in the following Examples was performed by an APTT measuring procedure.

1. Method

1.1) Specimen

- Control specimen group (NE): plasma (n = 30) with APTT being within the reference standard range (from 24 to 39 seconds) of Coagpia APTT-N (manufactured by Sekisui Medical Co., Ltd.).
- Subject specimen group (E1): plasma (n = 54) obtained from a patient with hemophilia A to whom emicizumab had been administered. The emicizumab concentration ranged from 12.2 to 79.2 $\mu$g/mL.
- Subject specimen group (E2): plasma (n = 15) obtained from a patient with hemophilia A who had an antibody (inhibitor) against the coagulation factor VIII (FVIII) and to whom emicizumab had been administered. The emicizumab concentration ranged from 12.0 to 64.0 μg/mL, and the inhibitor potency ranged from 0.8 to 202 BU/mL.

1.2) Coagulation reaction measurement

Coagpia APTT-N (manufactured by Sekisui Medical Co., Ltd.), which is a reagent for APTT measurement, was used as reagent for measurement, and Coagpia APTT-N calcium chloride solution (manufactured by Sekisui Medical Co., Ltd.) was used as a calcium chloride solution. The coagulation reaction of a sample containing a specimen was measured using the blood coagulation automatic analysis device CP3000 (manufactured by Sekisui Medical Co., Ltd.). 50μL of the specimen was heated in a cuvette at 37°C for 45 seconds, 50μL of the reagent for measurement at approximately 37°C was subsequently added, and after 171 seconds elapsed, 50μL of the calcium chloride solution was added, thus initiating the coagulation reaction. The reaction was performed at 37°C. In the measurement of the coagulation reaction, the cuvette was irradiated with light of a wavelength of 660 nm from a light source which is an LED, and the scattered light intensity of 90-degree side scattered light was measured at 0.1-second intervals. The measurement time was 360 seconds.

1.3) Acquisition of coagulation reaction curve

To the measured data of the coagulation reaction from each specimen, a smoothing process including denoising was applied, a zero adjustment process was applied so as to allow the scattered light intensity at the measurement initiation time point to be zero, and the coagulation reaction curve P(i) was created.

1.4) Detection of coagulation reaction end point Pe and determination of APTT

P(i) at the earliest time point when the accumulated ratio Z(i) of the coagulation reaction curve P(i) (see WO 2021/132552) becomes less than the accumulated ratio threshold Zs was detected as the coagulation reaction end point Pe. 1.001 was used as the accumulated ratio threshold Zs. A time point T(50) at which P(i) reached 50% of Pe was calculated, and was determined as APTT. The accumulated ratio Z(i) at a time point i was calculated as follows.

$$\texttt{Accumulated ratio Z(i) = Pb(i)/Pa(i)}$$

$$\texttt{Pa(i) = sum of P(i - 20) to P(i - 1)}$$

$$\texttt{Pb(i) = sum of P(i + 1) to P(i + 20)}$$

1.5) Calculation of T(X)

For each specimen, time T(X) at which P(i) reached X% of Pe detected in 1.4) was calculated based on the following equation. 100 items of X from 1 to 100 in increments of one were set, and T(1) to T(100) were calculated. Note that T(50) at X = 50 corresponded to APTT.

$$\texttt{P(T(X)) = Pe × X\%}$$

2. Coagulation reaction

Coagulation reaction curve P(i) in 2.1)

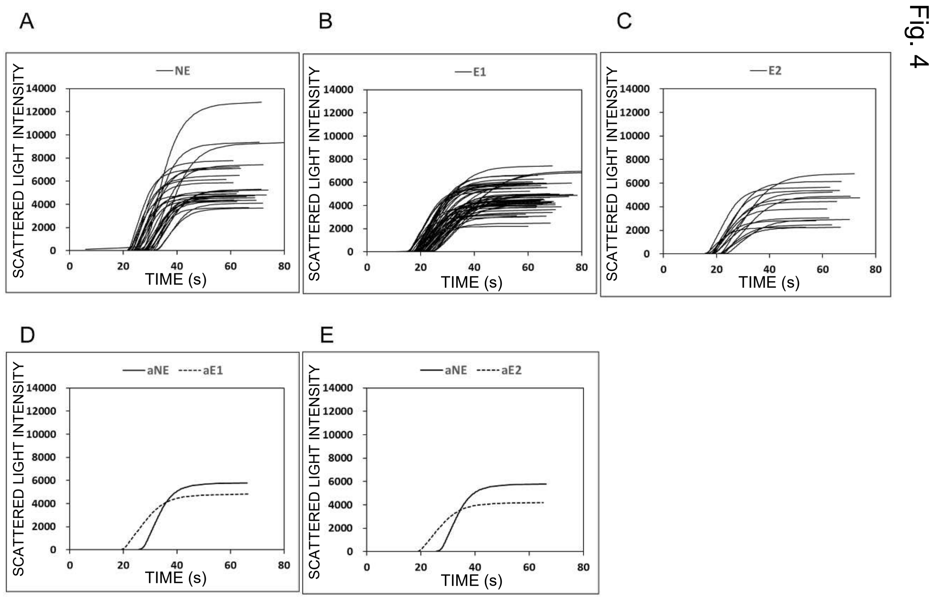

Fig. 4 illustrates the coagulation reaction curve P(i) of each specimen. The abscissa axis indicates time, and the ordinate axis indicates the coagulation reaction (scattered light intensity). Figs. 4A to 4C illustrate P(i) of specimens of NE, E1, and E2. Figs. 4D and 4E illustrate the averages (aE1 and aE2; dotted lines) of P(i) of E1 and E2 together with the average (aNE; solid line) of P(i) of NE. P(i) of emicizumab-administered groups (E1 and E2) had tendencies where the rise time of the reaction curve was rapid but the increase of the curve was gradual in comparison with the non-emicizumab-administered group (NE).

2.2) APTT calculation

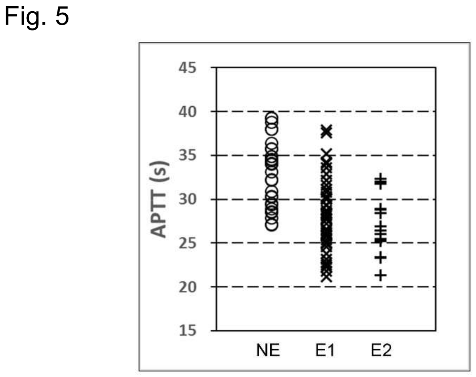

Fig. 5 illustrates APTT of NE, E1, and E2. APTT ranged from 27.1 to 39.3 seconds for NE, from 21.2 to 37.9 seconds for E1, and from 21.3 to 32.3 seconds for E2. The APTT distribution regions of E1 and E2 overlap the distribution region of NE. E1 and E2 exhibited APTT shorter than the lower limit (24 seconds) of the reference standard range in some cases. This result showed that the emicizumab-administered groups (E1 and E2), and the non-emicizumab-administered group (NE) cannot be distinguished from each other based only on

APTT.

2.3) Corrected reaction curve

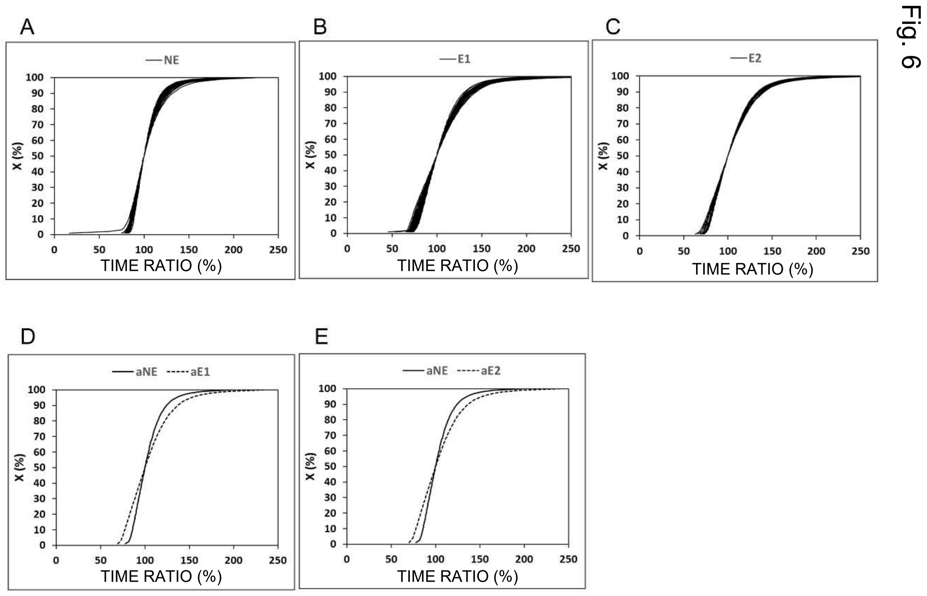

Fig. 6 illustrates data where the magnitude of the coagulation reaction of the specimen, and the reaction time are relativized. Hereinafter, this also called a corrected reaction curve. In Fig. 6, the abscissa axis indicates the time ratio $(T(X)/T(50))$ of $T(X)$ of each specimen, and the ordinate axis indicates X(%). Figs. 6A to 6C illustrate data of specimens of NE, E1, and E2. Figs. 6D and 6E illustrate the averages (aE1 and aE2; dotted lines) of the data of E1 and E2 together with the average (aNE; solid line) of NE. Similar to P(i) illustrated in Fig. 4, the emicizumab-administered groups (E1 and E2) have tendencies where the rise time of the corrected reaction curve was rapid but the increase of the curve was gradual in comparison with the non-emicizumab-administered group (NE).

3. Comparison of parameters between specimen groups

3.1) Example 1: Interval change rate of T(X)

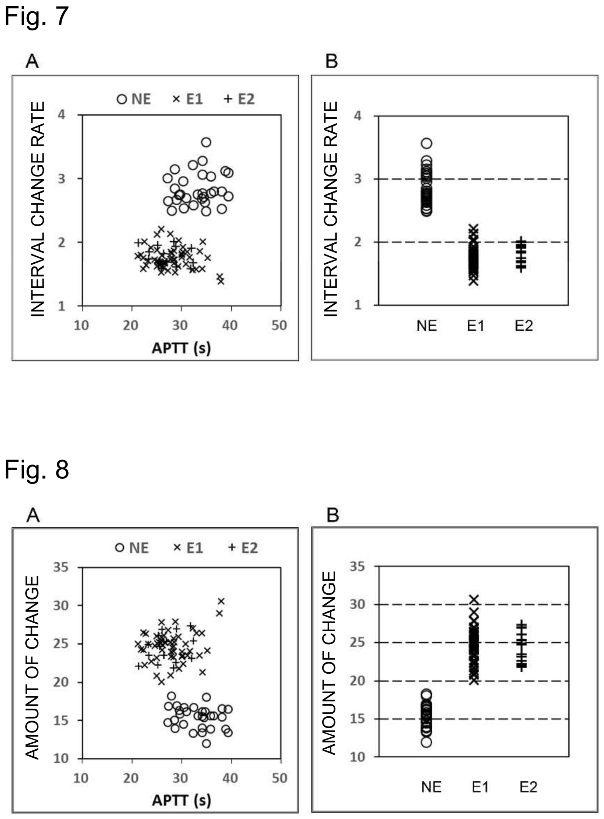

For each specimen, the interval change rate of $T(X)$ $[(X_2 - X_1)/\{(T(X_2)/T(50)) - (T(X_1)/T(50))\}]$ ($X_1 = 30$ and $X_2 = 50$) with X ranging from 30 to 50 was obtained. Fig. 7A illustrates a plot of the interval change rate of each specimen against APTT. Fig. 7B illustrates an interval change rate distribution of each specimen group. The interval change rates of E1 and E2 were smaller than that of NE. It was shown that the emicizumab-administered groups (E1 and E2) and the non-administered group (NE) could be distinguished from each other, based on the interval change rate of T(X).

3.2) Example 2: Amount of change of T(X)

For each specimen, the amount of change of $T(X)$ $[\{T(X_2) - T(X_1)\}/T(50)]$ ($X_1 = 30$ and $X_2 = 70$) with X ranging from 30 to 70 was obtained. Fig. 8A illustrates a plot of the amount of change of T(X) of each specimen against APTT. Fig. 8B illustrates the amount of change of T(X) of each specimen group. The amounts of change of T(X) of E1 and E2 were greater than that of NE. It was shown that the emicizumab-administered groups (E1 and E2) and the non-administered group (NE) could be distinguished from each other, based on the amount of change of T(X).

3.3) Example 3: Ratio of T(X)

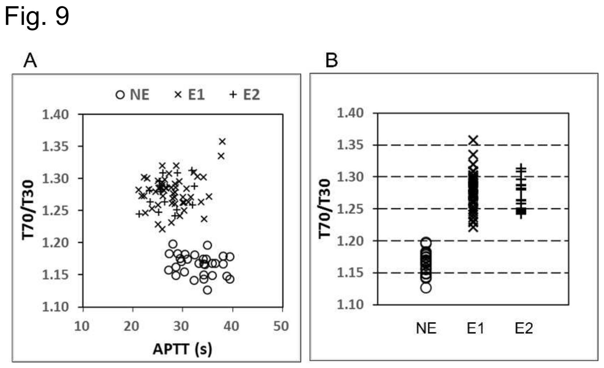

For each specimen, the ratio of $T(X)$ $[T(X_2)/T(X_1)]$ with X ranging from $X_1 = 30$ to $X_2 = 70$ was obtained. Fig. 9A illustrates a plot of T(70)/T(30) of each specimen against APTT. Fig. 9B illustrates a T(70)/T(30) distribution of each specimen group. T(70)/T(30) of E1 and E2 were larger than that of NE. It was shown that the emicizumab-administered groups (E1 and E2) and the non-administered group (NE) could be distinguished from each other, based on the T(X) ratio.

3.4) Example 4: Parameter about coefficient of variation CV of T(X)

$CV(X \pm 4)$ was obtained by the following equation.

$$CV(X \pm 4) = \{SD \text{ of } T(X - 4) \text{ to } T(X + 4)\}/\{AVE \text{ of } T(X - 4) \text{ to } T(X + 4)\} \times 100 \text{ [\%]}$$

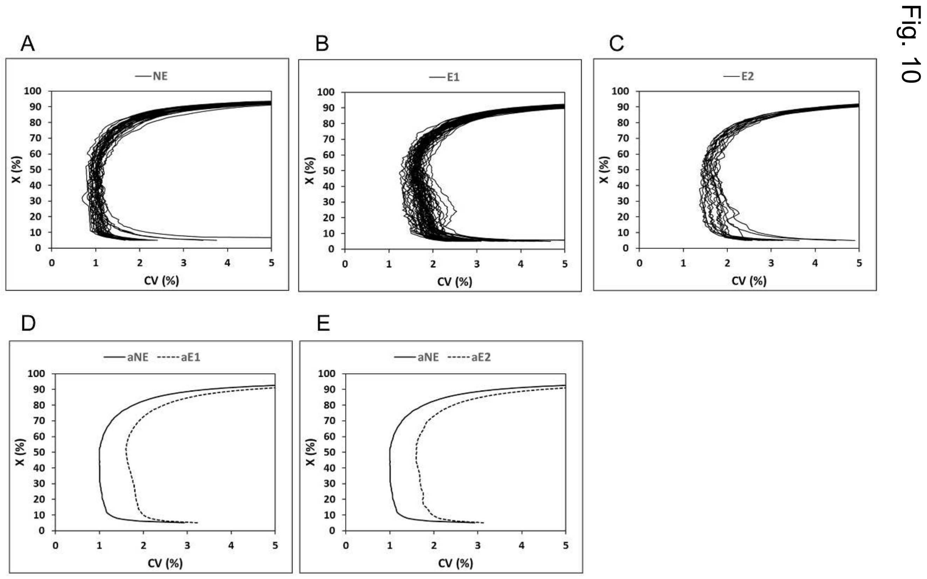

Figs. 10A to 10C illustrate the curve of the coagulation reaction of each of specimens of NE, E1, and E2, with the abscissa axis indicating $CV(X \pm 4)$ and the ordinate axis indicating X (%). Hereinafter, this is also called CV curves. Figs. 10D and 10E illustrate the averages (aE1 and aE2; dotted lines) of the CV curves of E1 and E2 together with the average (aNE; solid line) of NE. $CV(X \pm 4)$ of E1 and E2 were greater than that of NE.

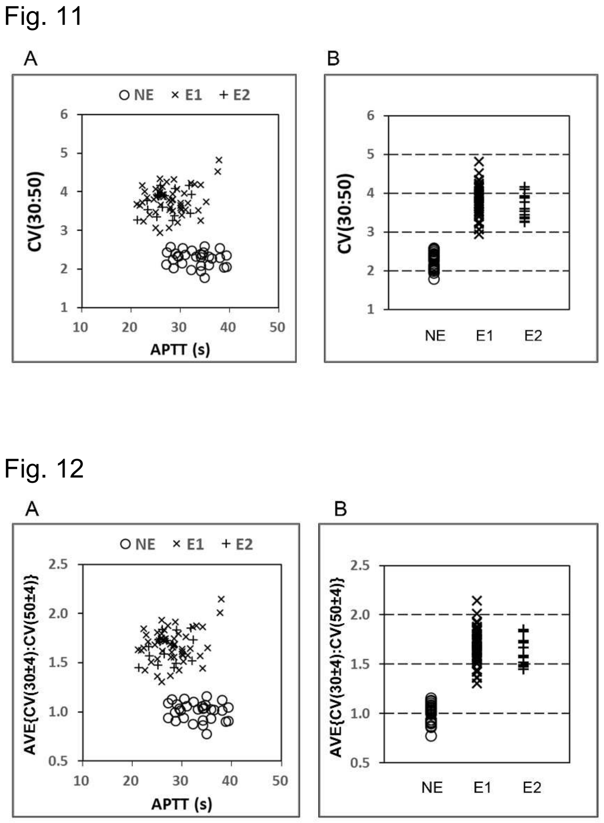

For each specimen, CV(30:50), which is the coefficient of variation of T(X), in a range of X = 30 to 50 was obtained. Fig. 11A illustrates a plot of CV(30:50) of each specimen against APTT. Fig. 11B illustrates a CV(30:50) distribution of each specimen group. CV(30:50) of E1 and E2 were greater than that of NE. It was shown that the emicizumab-administered groups (E1 and E2) and the non-administered group (NE) could be distinguished from each other, based on CV(30:50).

For each specimen, the average $AVE\{CV(30 \pm 4):CV(50 \pm 4)\}$ of $CV(X \pm 4)$ in the range of X = 30 to 50 was obtained. Fig. 12A illustrates a plot of $AVE\{CV(30 \pm 4):CV(50 \pm 4)\}$ of each specimen against APTT. Fig. 12B illustrates an $AVE\{CV(30 \pm 4):CV(50 \pm 4)\}$ distribution of each specimen group. $AVE\{CV(30 \pm 4):CV(50 \pm 4)\}$ of E1 and E2 were greater than that of NE. It was shown that the emicizumab-administered groups (E1 and E2) and the non-administered group (NE) could be distinguished from each other, based on AVE{CV(30 ± 4):CV(50 ± 4)}.

3.5) Comparative Example 1 Vmax

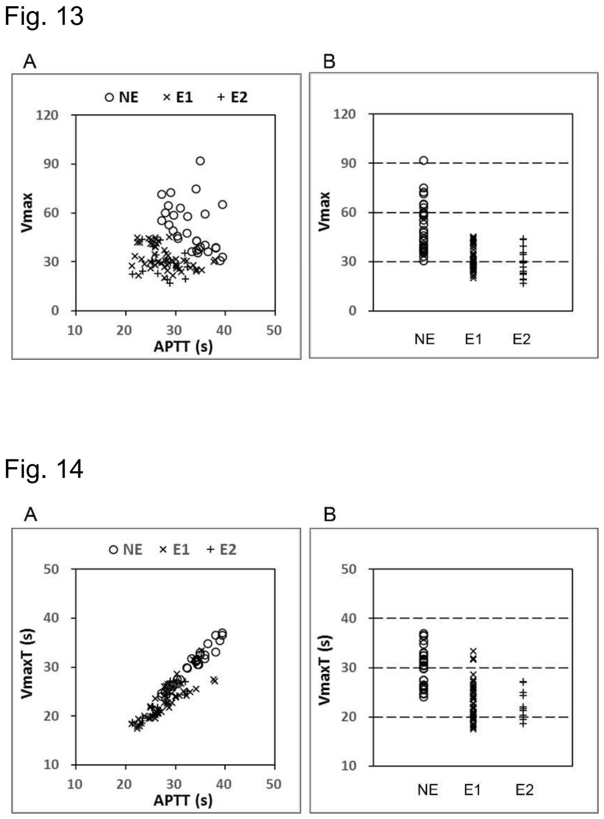

Fig. 13 illustrates distributions of the maximum Vmax (maximum rate) of the first derivative (reaction rate) of P(i) of NE, E1, and E2. Fig. 13A illustrates a plot of Vmax of each specimen against APTT. Fig. 13B illustrates a Vmax distribution of each specimen group. Vmax of E1 and E2 tended to be smaller than that of NE, but the distribution regions overlapped that of NE. The emicizumab-administered groups (E1 and E2), and non-administered group (NE) were unable to be distinguished from each other based only on Vmax.

3.6) Comparative Example 2 VmaxT

Fig. 14 illustrates a time VmaxT distribution where the first derivative (reaction rate) of P(i) of NE, E1, and E2 reaches Vmax. Fig. 14A illustrates a plot of VmaxT of each specimen against APTT. Fig. 14B illustrates a VmaxT distribution of each specimen group. While VmaxT of E1 and E2 tended to be smaller than that of NE, the distribution regions overlapped that of NE. The emicizumab-administered groups (E1 and E2), and non-administered group (NE) were unable to be distinguished from each other based only on VmaxT.

3.7) Example 5 SDI of parameter

The proximity of the parameter (P) between the emicizumab-administered groups (Emi; including E1 and E2) and the non-administered group (NE) was evaluated. The interval change rate of T(X) obtained in Example 1, the amount of change of T(X) obtained in Example 2, T(70)/T(30) obtained in Example 3, and CV(30:50) and AVE{CV(30 ± 4):CV(50 ± 4)} obtained in Example 4 were used as the parameter (P). The specimen with P being closest to Emi in NE (that is, P was the maximum or minimum) (NE proximal specimen), and the specimen with P being closest to NE in Emi (that is, P was the minimum or maximum) (Emi proximal specimen) were extracted. The absolute value of the standard deviation index (|SDI|) of P of each of the extracted two specimens with respect to the average of P in NE was calculated.

|SDI| = |{(NE proximal specimen or P of Emi proximal specimen) - (average of P in NE)}/(standard deviation of P in NE)|

Table 1 illustrates |SDI| of the NE proximal specimen and the Emi proximal specimen of each parameter. In the difference of |SDI| between the NE proximal specimen and the Emi proximal specimen, the fact that each parameter had a different distribution between Emi and NE was reflected, as illustrated in Examples 1 to 4. These results showed that using |SDI| of the parameter illustrated in Examples 1 to 4 could distinguish the emicizumab-administered group and the non-administered group from each other. For example, |SDI| of the parameter of the subject specimen with respect to the reference value was obtained, and was compared with the threshold, thereby allowing detecting whether the subject specimen is in the emicizumab-administered group or not. The average of the parameters of NE as described above can be used as the reference value. An intermediate value between |SDI| of the NE proximal specimen and |SDI| of the Emi proximal specimen as described above can be set as an example of the threshold.

[Table 1]

| Parameter (P) | A: |SDI| of P of NE proximal specimen | B: |SDI| of Emi proximal specimen | B-A | Intermediate value of A and B |
|---|---|---|---|---|
| Interval change rate of T(X) | 1.35 | 2.43 | 1.07 | 1.89 |
| Amount of change of T(X) | 1.94 | 2.11 | 0.17 | 2.02 |
| T70/T30 | 1.92 | 3.28 | 1.36 | 2.60 |
| CV(30:50) | 1.55 | 3.25 | 1.70 | 2.40 |
| AVE[CV(30±4):CV(30±4)] | 1.63 | 3.17 | 1.54 | 2.40 |

**Claims**

1. A method for analyzing a blood coagulation ability of a blood specimen, the method comprising:

1) detecting a coagulation reaction end point Pe of a coagulation reaction curve of a subject blood specimen in which presence or absence of a substance having coagulation factor VIII-substituting activity is required to be detected;

2) calculating T(X), where T(X) represents a measurement point or time at which the coagulation reaction curve reaches X% of Pe, and X is a variable greater than 0 and less than or equal to 100; and

3) detecting the subject blood specimen which contains the substance having coagulation factor VIII-substituting activity, based on T(X).

2. The method according to claim **1,** wherein the subject blood specimen is a blood specimen with no prolonged activated partial thromboplastin time (APTT).

3. The method according to claim 1,

wherein the 3) comprises calculating a parameter based on T(X), and detecting the subject blood specimen which contains the substance having coagulation factor VIII-substituting activity, based on the parameter, and

the parameter is at least one selected from the group consisting of an amount of change of T(X), a ratio of T(X), an interval change rate of T(X), and a parameter about a coefficient of variation of T(X) in a given range of X, and standard deviation indices (SDI) thereof.

4. The method according to claim 3,

wherein the amount of change of T(X) in the given range of X is $[(T(X_2)/C) - (T(X_1)/C)]$,

the ratio of T(X) in the given range of X is $[T(X_2)/T(X_1)]$, and

the interval change rate of T(X) in the given range of X is $[(X_2 - X1)/\{(T(X_2)/C) - (T(X_1)/C)\}]$,

where $X_1$ and $X_2$ independently range from 1 to 100, $X_2 > X_1$, and C is a constant.

5. The method according to claim 3,

wherein the parameter about the coefficient of variation of T(X) is $CV(X_1:X_2)$, $CV(X_1 - J:X_1 + J)$, or $AVE\{CV(X_1 \pm J):CV(X_2 \pm J)\}$,

where

$CV(X_1:X_2)$ is the coefficient of variation of T(X) which is in a range of X from $X_1$ to $X_2$ and is represented by the following equations,

$CV(X_1:X_2) = SD\{T(X_1):T(X_2)\}/AVE\{T(X_1):T(X_2)\} \times 100$ [%],

$AVE\{T(X_1):T(X_2)\}$: an average of $T(X_1)$ to $T(X_2)$,

$SD\{T(X_1):T(X_2)\}$: a standard deviation of $T(X_1)$ to $T(X_2)$,

$CV(X_1 - J:X_1 + J)$ is a coefficient of variation of T(X) in a range from $(X_1 - J)$ to $(X_1 + J)$, and

$AVE\{CV(X_1 \pm J):CV(X_2 \pm J)\}$ is an average of CV(X - J:X + J) with X ranging from $X_1$ to $X_2$,

where $X_1$ and $X_2$ independently range from 1 to 100, $X_2 > X_1$, and J = 2 to 44.

6. The method according to claim 3, further comprising detecting the subject blood specimen which contains the substance having coagulation factor VIII-substituting activity, by comparing the parameter based on T(X) with a given threshold.

7. The method according to claim 1, further comprising outputting a result of the detecting.

8. The method according to claim 1, further comprising:

measuring the activated partial thromboplastin time (APTT) of a blood specimen; and

selecting the blood specimen with no prolonged APTT, as the subject blood specimen in which the presence or absence of the substance having coagulation factor VIII-substituting activity is required to be detected.

9. The method according to claim **1,** wherein the substance having coagulation factor VIII-substituting activity is a bispecific antibody substituting for a cofactor function of coagulation factor VIII.

10. The method according to claim **1,** wherein the substance having coagulation factor VIII-substituting activity is emicizumab.

11. A program for implementing the method according to claim **1.**

12. A device for implementing the method according to claim **1.**

13. A system for implementing the method according to claim **1,** the system comprising: a program for implementing the method according to claim **1;** and a device controlled by the program.

14. The device according to claim 12 or the system according to claim 13, further comprising an output unit for outputting a result of the detecting of the subject blood specimen which contains the substance having coagulation factor VIII-substituting activity by the method according to claim **1.**

Fig. 1

Fig. 2

Fig. 3A

ACQUIRE P(i)  S1

CALCULATE APTT FROM P(i)  S2

COMPARE
APTT WITH UPPER LIMIT OF
REFERENCE RANGE  S3

APTT > UPPER LIMIT

APTT ≤ UPPER LIMIT

CALCULATE T(X) FROM P(i)  S4

CALCULATE ONE OR MORE
PARAMETERS FROM T(X)  S5

COMPARE
PARAMETER(S) WITH
THRESHOLD  S6

NONE SATISFY
THRESHOLD

AT LEAST ONE SATISFIES THRESHOLD

OUTPUT APTT CALCULATION RESULT TO
WHICH FVIII-SUBSTITUTING SUBSTANCE
DETECTION INFORMATION IS ADDED  S7

NORMAL OUTPUT OF APTT
CALCULATION RESULT  S8

Fig. 3B

Fig. 4

Fig. 5

Fig. 6

Fig. 7

Fig. 8

Fig. 9

Fig. 10

Fig. 11

Fig. 12

26

Fig. 13

Fig. 14

## INTERNATIONAL SEARCH REPORT

| International application No. |
|---|
| **PCT/JP2023/047188** |

**A. CLASSIFICATION OF SUBJECT MATTER**

***G01N 33/86***(2006.01)i
FI: G01N33/86

According to International Patent Classification (IPC) or to both national classification and IPC

**B. FIELDS SEARCHED**

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2022/186381 A1 (SEKISUI MEDICAL CO., LTD.) 09 September 2022 (2022-09-09) paragraphs [0010]-[0057] | 1-14 |
| A | WO 2016/047652 A1 (NARA MEDICAL UNIVERSITY) 31 March 2016 (2016-03-31) paragraphs [0009], [0016], [0073]-[0094] | 1-14 |
| A | WO 2014/050926 A1 (CHUGAI SEIYAKU KABUSHIKI KAISHA) 03 April 2014 (2014-04-03) paragraphs [0007], [0011], [0042]-[0048] | 1-14 |
| A | WO 2016/170944 A1 (NARA MEDICAL UNIVERSITY) 27 October 2016 (2016-10-27) paragraphs [0009]-[0011], [0118]-[0154] | 1-14 |
| A | WADA, Hideo. A Clot Waveform Analysis of Thrombin Time Using a Small Amount of Thrombin Is Useful for Evaluating the Clotting Activity of Plasma Independent of the Presence of Emicizumab. J. Clin. Med. 2022, 11,6142, pp. 1-10, https://doi.org/10.3390/jcm11206142 p. 1, abstract, pp. 7-9, discussion, fig. 7 | 1-14 |

☑ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 March 2024** | **19 March 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2023/047188** |

**C. DOCUMENTS CONSIDERED TO BE RELEVANT**

| Category* | Citation of document, with indication, where appropriate, of the relevant passages | Relevant to claim No. |
| --- | --- | --- |
| A | HATAYAMA, Yuki. Regression Analysis to Estimate the Factor VIII Activity of Patients with Hemophilia A Without Inhibitor who Received Emicizumab Therapy. Clinical and Applied Thrombosis/Hemostasis. 2022, vol. 28, pp. 1-6, DOI: 10.1177/10760296221082992<br>p. 1, abstract, p. 3, left column, lines 4-21, fig. 2 | 1-14 |
| A | 加藤　小波, 凝固第VIII因子定量試薬「レボヘムFVIII合成基質」の基礎的検討及び凝固一段法の比較, 医学検査, 2022, vol. 71, no. 1, pp. 66-72, DOI: 10.14932/jamt.21-5, (KATO, Konami. Basic study of coagulation factor VIII quantification reagent "Revohem FVIII synthetic substrate" used in chromogenic assay and comparison of chromogenic assay with the one-stage assay. Japanese Journal of Medical Technology.)<br>entire text, all drawings | 1-14 |
| A | 粟根　舞, エミシズマブ投与患者における凝固波形解析の評価, 第69回日本臨床検査医学会学術集会, 25 October 2022, vol. 70, O-151, p. 201, (The 69th Annual Meeting of Japanese Society of Laboratory Medicine), non-official translation (KURINE, Mai. Evaluation of coagulation waveform analysis in emicizumab-treated patients.)<br>entire text, all drawings | 1-14 |
| P, X | 前田　佑華, APTT凝固波形解析によるEmicizumab含有検体の鑑別法の開発, 日本検査血液学会雑誌, 06 July 2023, vol. 24, academic conference issue O18-1, S154, (MAEDA, Yuka. Journal of the Japanese Society for Laboratory Hematology.), non-official translation (Development of a method for differentiating emicizumab-containing samples using APTT coagulation waveform analysis)<br>entire text, all drawings | 1-14 |

Form PCT/ISA/210 (second sheet) (July 2022)

**INTERNATIONAL SEARCH REPORT**
Information on patent family members

International application No.

**PCT/JP2023/047188**

| Patent document cited in search report | | | Publication date (day/month/year) | Patent family member(s) | | | Publication date (day/month/year) |
|---|---|---|---|---|---|---|---|
| WO | 2022/186381 | A1 | 09 September 2022 | CN | 116917739 | A | |
| WO | 2016/047652 | A1 | 31 March 2016 | US | 2018/0011114 | A1 | |
| | | | | paragraphs [0011], [0043]-[0045], [0161]-[0183] | | | |
| | | | | EP | 3199952 | A1 | |
| | | | | CN | 106716139 | A | |
| | | | | KR | 10-2017-0084030 | A | |
| WO | 2014/050926 | A1 | 03 April 2014 | US | 2015/0240287 | A1 | |
| | | | | paragraphs [0008], [0018], [0068]-[0078] | | | |
| | | | | EP | 2902787 | A1 | |
| | | | | KR | 10-2015-0068406 | A | |
| | | | | CN | 104937423 | A | |
| WO | 2016/170944 | A1 | 27 October 2016 | US | 2018/0045709 | A1 | |
| | | | | paragraphs [0010]-[0012], [0254]-[0291] | | | |
| | | | | EP | 3287791 | A1 | |
| | | | | CN | 107533071 | A | |

Form PCT/ISA/210 (patent family annex) (July 2022)

**EP 4 644 906 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014050926 A
- WO 2016170944 A
- JP 2017106925 A
- WO 2021206107 A
- WO 2021132552 A
- JP H6249855 A
- WO 2021177452 A